# EUROPÄISCHE PATENTSCHRIFT

(11) **EP 0 370 325 B1**
(45) Veröffentlichungstag und Bekanntmachung des Hinweises auf die Patenterteilung: **09.06.1993**
(21) Anmeldenummer: 89120825.8
(22) Anmeldetag: 10.11.1989
(51) Int. Cl.: C07B 31/00, C07C 309/46, C07C 303/22

(54) **Verfahren zur reduktiven Enthalogenierung von Aromaten**
Process for the reductive dehalogenation of aromatics
Procédé pour la déshalogénation réductive de composés aromatiques

(30) Priorität: 22.11.1988 DE 3839329
(43) Veröffentlichungstag der Anmeldung: 30.05.1990
(73) Patentinhaber: BASF Aktiengesellschaft, 67063 Ludwigshafen (DE)
(72) Erfinder: Eilingsfeld, Heinz, Dr., D-6710 Frankenthal (DE); Siegel, Bernd, Dr., D-6700 Ludwigshafen (DE); Patsch, Manfred, Dr., D-6706 Wachenheim (DE)

(56) Entgegenhaltungen:
- GB-A- 1 458 633
- SYNTHESIS, Nr. 2, Februar 1988, pages 91-95, Georg Thieme Verlag, Stuttgart, DE; S. RAM: "Ammonium Formate in Organic Synthesis: A Versatile Agent in Catalytic Hydrogen Transfer Reductions"
- CHEMICAL ABSTRACTS, Band 80, Nr. 1, 7. Januar 1974, Seite 281, Zusammenfassung Nr. 3230n, Columbus, Ohio, US; & JP-A-73 61 402 (YONEYAMA CHEMICALS CO. LTD) 28-08-1973

## Beschreibung

Die vorliegende Erfindung betrifft ein neues Verfahren zur reduktiven Enthalogenierung von Chlor- oder Bromaromaten oder -heteroaromaten, die jeweils durch Hydroxy, Carboxyl und/oder Hydroxysulfonyl substituiert sind und auch noch weitere Substituenten aufweisen können, durch Behandlung dieser Verbindungen mit Raney-Nickel und Alkaliformiat in wäßrig-alkalischem Medium bei einer Temperatur von 80 bis 250°C und bei einem Druck von 1 bis 100 bar.

Die GB-A-1 458 633 lehrt die Enthalogenierung von Chlorpyridin sowie von verschiedenen Chlor- und Brombenzolen mittels eines Palladium/Kohle-Katalysators und Natriumformiat in wäßrig-alkalischem Milieu, wobei zusätzlich in Gegenwart eines Phasentransferkatalysators gearbeitet werden muß.

Weiterhin beschreibt Synthesis 1988, 91, daß man chlorhaltige Biphenyle oder 2,4,6-Trichlorphenol mittels eines Katalysators auf Basis Palladium/Kohle in Gegenwart von Ammoniumformiat und in Methanol oder Essigsäure als Lösungsmittel in die entsprechenden chlorfreien Verbindungen überführen kann. In dieser Veröffentlichung wird auch erwähnt, daß man 2-Iod-4-nitrobenzamid mittels Raney-Nickel und Ammoniumformiat in 4-Aminobenzamid umwandeln könne. Nähere Einzelheiten dazu sind jedoch nicht angegeben.

In Chem. Abstracts, Band 80, 1974, Zusammenfassung Nr. 3230n ist die Enthalogenierung von p-Chlorbenzoesäure mittels eines 10%igen Palladium/Kohle-Katalysators und Natriumformiat in wäßriger Natronlauge bei 95-100° C in 82%iger Ausbeute offenbart.

Aus Synthesis 1980, 425, ist nun auch bekannt, daß die hydrogenolytische Abspaltung von Halogen aus organischen Halogenverbindungen speziell bei Iodverbindungen sehr leicht verläuft, wogegen die anderen Halogenverbindungen (Fluor-, Chlor- und Bromverbindungen) viel schwieriger einer hydrogenolytischen Spaltung zu unterwerfen sind.

Aufgabe der vorliegenden Erfindung war es nun, ein Verfahren zur Entchlorierung und Entbromierung von Aromaten und Heteroaromaten bereitzustellen, bei dem die Zielprodukte in einfacher Weise, ohne großen technischen Aufwand und in guter Ausbeute anfallen sollten, wobei möglichst auf kostspielige Palladiumkatalysatoren, Phasentransferkatalysatoren sowie organische Lösungsmittel verzichtet werden sollte.

Aufgrund des Standes der Technik war nun zu erwarten, daß die Entchlorierung oder die Entbromierung solcher Verbindungen mit anderen Metallkatalysatoren, die sich von Metallen ableiten, die weniger edel als Palladium sind, unter diesen Bedingungen entweder überhaupt nicht gelingen oder allenfalls die Zielprodukte in unbefriedigender Ausbeute liefern würde.

Überraschenderweise wurde jedoch gefunden, daß die reduktive Enthalogenierung von Halogenverbindungen der Formel I

R(Hal)ₙ (I)

zu halogenfreien Verbindungen der Formel II

R(H)ₙ (II),

wobei n jeweils 1 bis 3, Hal Chlor oder Brom und R jeweils einen aromatischen oder heteroaromatischen Rest, der durch Hydroxy, Carboxyl und/oder Hydroxysulfonyl substituiert ist und noch weitere Substituenten aufweisen kann, bedeuten, durch Behandlung der Halogenverbindungen der Formel I mit einem Katalysator, enthaltend ein Metall der Gruppe 8 des Periodensystems der Elemente, und mit einem Alkaliformiat in wäßrig-alkalischem Medium bei einer Temperatur von 80 bis 250°C und bei einem Druck von 1 bis 100 bar vorteilhaft gelingt, wenn man die Behandlung mit Raney-Nickel als Katalysator durchführt.

Unter aromatischen oder heteroaromatischen Systemen, die den entsprechenden Chlor- oder Bromverbindungen der Formel I zugrundeliegen, sind erfindungsgemäß solche zu verstehen, die sich ableiten von Benzol oder dessen ein- oder mehrfach benzoanellierten Produkten oder von 5- oder 6-gliedrigen aromatischen Heterocyclen, die 1 bis 3 gleiche oder verschiedene Heteroatome aus der Gruppe, bestehend aus Stickstoff, Sauerstoff und Schwefel, aufweisen und gegebenenfalls noch benzoanelliert sind.

Einzelne aromatische oder heteroaromatische Grundkörper, die den halogenhaltigen Chlor- oder Bromverbindungen der Formel I zugrundeliegen, sind z.B. Benzol, Naphthalin, Anthracen, Phenanthren, Pyrrol, Pyrazol, Imidazol, Furan, Oxazol, Isoxazol, Thiazol, Isothiazol, Oxdiazol, Thiadiazol, Pyridin, Pyridazin, Pyrimidin, Pyrazin, Indol, Indazol, Benzimidazol, Benzofuran, Benzothiophen, Benzoxazol, Benzthiazol, Chinolin, Isochinolin, Phthalazin, Chinoxalin, Chinazolin oder Phenazin.

Wie oben bereits ausgeführt, weisen die halogenhaltigen Verbindungen der Formel I 1 bis 3 Chlor- und/oder Bromatome auf und sind durch Hydroxy, Carboxyl und/oder Hydroxysulfonyl substituiert. Wenn noch weitere Substituenten im Molekül vorhanden sind, so kommen dafür z.B. Amino, Cyano oder gegebenenfalls durch C₁-C₄-Alkyl oder C₁-C₄-Alkoxy substituiertes Benzoyl in Betracht. Bei Anthracenderivaten sind auch die jeweiligen Anthrachinonderivate geeignet.

Vorzugsweise wird das erfindungsgemäße Verfahren mit solchen Halogenverbindungen der Formel I durchgeführt, in der R für einen Phenyl-, Naphthyl-, Anthroyl-, Phenanthroyl-, Pyridyl-, Indolyl- oder Chinolinylrest steht, wobei dieser Rest jeweils durch Hydroxy, Carboxyl und/oder Hydroxysulfonyl substituiert ist und noch weitere Substituenten aufweisen kann. Dabei ist der entsprechend substituierte Phenylrest besonders hervorzuheben.

In einer weiteren bevorzugten Ausführungsform werden solche Halogenverbindungen der Formel I verwendet, in der Hal Chlor und n 1 oder 2 und insbesondere 1 bedeuten.

Bevorzugt ist weiterhin eine Verfahrensweise, in der man Halogenverbindungen der Formel I, in der R einen aromatischen oder heteroaromatischen Rest, der 1 oder 2 Hydroxy-, Carboxyl- und/oder Hydroxysulfonylreste aufweist, umsetzt.

Besonders wichtige Benzolderivate, die im erfindungsgemäßen Verfahren zur Anwendung kommen, sind solche, die ein Chloratom aufweisen und außer einer Hydroxysulfonylgruppe auch noch eine Aminogruppe tragen.

Als Katalysator im neuen Verfahren dient, wie oben bereits ausgeführt, Raney-Nickel. Dabei können die handelsüblichen Produkte verwendet werden.

Alkaliformiate, die im erfindungsgemäßen Verfahren zur Anwendung kommen, sind beispielsweise Lithiumformiat, Natriumformiat oder Kaliumformiat. Die Verwendung von Natriumformiat ist bevorzugt.

Erfindungsgemäß wird die Enthalogenierung in alkalisch wäßrigem Medium vorgenommen. Als solche Reaktionsmedien sind z.B. wäßrige Lösungen von Alkalihydroxiden, wie Lithiumhydroxid, Natriumhydroxid oder Kaliumhydroxid, zu nennen, wobei die Verwendung einer wäßrigen Natriumhydroxidlösung bevorzugt ist. Die Konzentration an Alkalihydroxid beträgt in der Regel 1 bis 15 Gew.%, vorzugsweise 5 bis 10 Gew.%, jeweils bezogen auf das Gewicht der Lösung.

Die Temperatur, bei der die erfindungsgemäße Umsetzung durchgeführt wird, liegt in der Regel bei 80 bis 250°C, vorzugsweise 100 bis 200°C und insbesondere 130 bis 160°C, wobei der Temperaturbereich, der bei ca. 140°C liegt, ganz besonders hervorzuheben ist.

Das neue Verfahren wird im allgemeinen bei einem Druck von 1 bis 100 bar, vorzugsweise 1 bis 50 bar, insbesondere 1 bis 10 bar und ganz besonders 1 bis 5 bar vorgenommen.

Das erfindungsgemäße Verfahren wird zweckmäßig so durchgeführt, daß man die Halogenverbindung I zusammen mit Raney-Nickel und dem Alkaliformiat in das wäßrig alkalische Medium gibt und die Reaktionsmischung dann unter Rühren auf die erfindungsgemäße Temperatur erhitzt. Führt man die Umsetzung nicht bei atmosphärischem Druck, sondern in einem höheren Druckbereich durch, so arbeitet man dabei in der Regel in einem Autoklaven oder in einer anderen Druckapparatur. Wenn die Enthalogenierung beendet ist, was im allgemeinen 3 bis 30 Stunden in Anspruch nimmt, wird abgekühlt, gegebenenfalls entspannt und das Raney-Nickel abfiltriert.

Die so erhaltene Reaktionslösung wird dann mit Säure, im allgemeinen mit Mineralsäure, z.B. Salzsäure oder Schwefelsäure, behandelt. Dabei wird ein pH-Wert eingestellt, der im neutralen oder im sauren Bereich (pH 1 bis 7) liegt. Das Zielprodukt der Formel II fällt unter diesen Bedingungen in der Regel als Niederschlag aus und kann dann, z.B. mittels Filtration, abgetrennt, gewaschen und getrocknet werden.

Raney-Nickel wird im erfindungsgemäßen Verfahren in der Regel in einer Menge von 1 bis 100 Gew.%, vorzugsweise 10 bis 60 Gew.% und insbesondere 20 bis 40 Gew.%, jeweils bezogen auf das Gewicht der Halogenverbindung der Formel I, verwendet. Es hat sich gezeigt, daß die Katalysatormenge abhängig von der Reaktionstemperatur ist. Je höher die Reaktionstemperatur gewählt wird, desto niedriger ist die benötigte Menge an Raney-Nickel.

Alkaliformiat wird üblicherweise in einer Menge von 1 bis 15 Gew.%, vorzugsweise 4 bis 12 Gew.%, jeweils bezogen auf das Gewicht der Lösung, angewandt. Es ist natürlich auch möglich, anstelle der Alkaliformiate Ameisensäure selbst zu verwenden, da sich in diesem Fall die Alkaliformiate zwangsläufig durch Reaktion mit der wäßrigen Alkalihydroxidlösung bilden.

Das erfindungsgemäße Verfahren kann sowohl in kontinuierlicher als auch diskontinuierlicher Arbeitsweise durchgeführt werden.

Im neuen Verfahren fallen die Zielprodukte im allgemeinen in guter Ausbeute und hoher Reinheit an. Dies ist überraschend, da in den eingangs abgehandelten bekannten Verfahren in den meisten Fällen die Bildung von unerwünschten Nebenprodukten in beträchtlichem Umfang beobachtet wird.

Die mittels des neuen Verfahrens erhältichen Verbindungen sind wertvolle Zwischenprodukte für die Synthese von Farbstoffen.

Die folgenden Beispiele sollen die Erfindung näher erläutern.

### Beispiel 1

104 g 5-Chlor-2-aminobenzolsulfonsäure wurden in 1000 ml Wasser mit 160 g 50gew.%iger Natronlauge, 34 g Natriumformiat und 37,5 g Raney-Nickel versetzt und 16 Stunden im Autoklaven auf eine Temperatur von 150°C erhitzt (4,5 bar Innendruck). Nach dem Abkühlen wurde die Reaktionslösung vom Nickel abgetrennt, mit verdünnter Salzsäure auf einen pH-Wert von 4 gestellt und eingedampft. Es wurden 260 g eines Feststoffs isoliert, der zu 33,3% aus 2-Aminobenzolsulfonsäure (Ausbeute: 100% d. Th.) und 66,7% Natriumchlorid bestand.

### Beispiel 2

62,3 g 5-Chlor-2-aminobenzolsulfonsäure, 1000 ml Wasser, 72 g 50gew.%ige Natronlauge, 20,4 g Natriumformiat und 50 g Raney-Nickel wurden 16 Stunden lang zum Sieden erhitzt (Temperatur: 100°C). Das Reaktionsgemisch wurde heiß abfiltriert und die abgekühlte Lösung mittels verdünnter Salzssäure auf pH 1 gestellt. Der resultierende Niedersachlag wurde abgesaugt, gewaschen und getrocknet. Gesamtausbeute 46,5 g 2-Aminobenzolsulfonsäure (89,6% d. Th.).

### Beispiel 3 (Vergleich)

207,5 g 5-Chlor-2-amino-benzolsulfonsäure wurden mit 1000 ml Wasser, 240 g 50gew.%iger Natronlauge, 68 g Natriumformiat und 6 g Palladium/Kohle (10gew.%ig) 6 Stunden unter Rückfluß erhitzt. Das Reaktionsgemisch wurde dann heiß filtriert und auf Raumtemperatur abgekühlt und mit verdünnter Salzsäure auf pH 1 gestellt. Der Niederschlag wurde abgesaugt, gewaschen und getrocknet. Ausbeute: 170 g eines Gemisches, das zu 61,6% aus 2-Aminobenzolsulfonsäure und 38,4% aus Nebenprodukten bestand.

### Beispiel 4

104 g 5-Chlor-2-aminobenzolsulfonsäure wurden in 1000 ml Wasser mit 160 g 50gew.%iger Natronlauge, 34 g Natriumformiat und 25 g Raney-Nickel versetzt und 16 Stunden im Autoklaven auf eine Temperatur von 185°C (10 bar Innendruck) erhitzt. Nach dem Abkühlen wurde die Lösung vom Nickel abgetrennt, mit verdünnter Salzsäure auf einen pH-Wert von 4 gestellt und eingedampft. Es wurden 220 g eines Feststoffs isoliert, der zu 33,8% aus 2-Aminobenzolsulfonsäure (Ausbeute: 85,6% d. Th.), 1,4% 5-Chlor-2-aminobenzolsulfonsäure und 64,8% Natriumchlorid bestand.

### Beispiel 5

181 g 6-Chlor-3,5-diaminobenzolsulfonsäure (61,5 gew.%ig) wurden mit 1000 ml Wasser, 160 g 50 gew.%iger Natronlauge, 34 g Natriumformiat und 100 g Raney-Nickel im Autoklaven 16 Stunden auf 140°C (3,5 bar Innendruck) erhitzt. Nach dem Abkühlen wurde das Nickel abgetrennt und die Reaktionslösung mit verdünnter Salzsäure auf einen pH-Wert von 4 gestellt und eingedampft. Es wurden 267 g eines Feststoffs isoliert, der zu 35,2% aus 3,5-Diaminobenzolsulfonsäure (Ausbeute: 100% d. Th.) und zu 64,8% aus Kochsalz bestand.

### Beispiel 6

Beispiel 6 wurde analog Beispiel 5 durchgeführt, jedoch wurde 4-Chlor-3,5-diaminobenzolsulfonsäure verwendet.

## Patentansprüche

1. Verfahren zur reduktiven Enthalogenierung von Halogenverbindungen der Formel I
R(Hal)ₙ (I)
zu halogenfreien Verbindungen der Formel II
R(H)ₙ (II),
wobei n jeweils 1 bis 3, Hal Chlor oder Brom und R jeweils einen aromatischen oder heteroaromatischen Rest, der durch Hydroxy, Carboxyl und/oder Hydroxysulfonyl substituiert ist und noch weitere Substituenten aufweisen kann, bedeuten, durch Behandlung der Halogenverbindungen der Formel I mit einem Katalysator, enthaltend ein Metall der Gruppe 8 des Periodensystems der Elemente, und mit einem Alkaliformiat in wäßrig-alkalischem Medium bei einer Temperatur von 80 bis 250°C und bei einem Druck von 1 bis 100 bar, dadurch gekennzeichnet, daß man die Behandlung mit Raney-Nickel als Katalysator durchführt.

2. Verfahren gemäß Anspruch 1, dadurch gekennzeichnet, daß man die Behandlung bei einer Temperatur von 100 bis 200°C durchführt.

3. Verfahren gemäß Anspruch 1, dadurch gekennzeichnet, daß man die Behandlung bei einem Druck von 1 bis 50 bar durchführt.

4. Verfahren gemäß Anspruch 1, dadurch gekennzeichnet, daß Hal Chlor bedeutet.

5. Verfahren gemäß Anspruch 1, dadurch gekennzeichnet, daß R für einen Phenyl-, Naphthyl-, Anthroyl-, Phenanthroyl-, Pyridyl-, Indolyl- oder Chinolinylrest steht, wobei dieser Rest jeweils durch Hydroxy, Carboxyl und/oder Hydroxysulfonyl substituiert ist und noch weitere Substituenten aufweisen kann.

## Claims

1. A process for the reductive dehalogenation of halogen compounds of the formula I
R(Hal)n (I)
to give halogen-free compounds of the formula II
R(H)n (II)
where n is in each case from 1 to 3, Hal is chlorine or bromine and R is in each case an aromatic or heteroaromatic radical which is substituted by hydroxyl, carboxyl and/or hydroxysulfonyl and may contain further substituents, by treating the halogen compounds of the formula I with a catalyst containing a metal from group 8 of the Periodic Table of the Elements and with an alkali metal formate in an aqueous-alkaline medium at from 80 to 250°C and at from 1 to 100 bar, which comprises carrying out the treatment using a Raney nickel as the catalyst.

2. A process as claimed in claim 1, wherein the treatment is carried out at from 100 to 200°C.

3. A process as claimed in claim 1, wherein the treatment is carried out at from 1 to 50 bar.

4. A process as claimed in claim 1, wherein Hal is chlorine.

5. A process as claimed in claim 1, wherein R is phenyl, naphthyl, anthroyl, phenanthroyl, pyridyl, indolyl or quinonyl, where each of these radicals is substituted by hydroxyl, carboxyl and/or hydroxysulfonyl and may contain further substituents.

## Revendications

1. Procédé de déshalogénation réductive de composés halogénés de formule I
R(Hal)ₙ (I)
en composés dénués d'halogène de formule II
R(H)ₙ (II)
n représentant chaque fois un nombre de 1 à 3, Hal un atome de chlore ou de brome et R représentant chaque fois un reste aromatique ou hétéroaromatique qui est substitué par des groupements hydroxy, carboxyle et/ou hydroxysulfonyle et peut encore porter d'autres substituants, par traitement des composés halogénés de formule I par un catalyseur contenant un métal du groupe VIII du système périodique et par un formiate alcalin, dans un milieu hydro-alcalin à une température de 80 à 250°C et sous une pression de 1 à 100 bar, caractérisé en ce qu'on effectue le traitement avec du nickel de Raney comme catalyseur.

2. Procédé selon la revendication 1, caractérisé en ce qu'on effectue le traitement à une température de 100 à 200°C.

3. Procédé selon la revendication 1, caractérisé en ce qu'on effectue le traitement sous une pression de 1 à 50 bar.

4. Procédé selon la revendication 1, caractérisé en ce que Hal représente un atome de chlore.

5. Procédé selon la revendication 1, caractérisé en ce que R est mis pour un reste phényle, naphtyle, anthroyle, phénanthroyle, pyridyle, indolyle ou quinolyle, chacun de ces restes étant substitué par des groupements hydroxy, carboxyle et/ou hydroxysulfonyle et pouvant encore porter d'autres substituants.
